(19) Europäisches Patentamt
European Patent Office
Office européen des brevets

(11) Veröffentlichungsnummer: **0 201 031**
**B1**

(12) # EUROPÄISCHE PATENTSCHRIFT

(45) Veröffentlichungstag der Patentschrift: **02.08.89**

(21) Anmeldenummer: **86105902.0**

(22) Anmeldetag: **29.04.86**

(51) Int. Cl.⁴: **C 07 C 69/653**, C 07 C 125/065, A 61 K 6/08

(54) **(Meth)-Acrylsäureester und ihre Verwendung.**

(30) Priorität: **07.05.85 DE 3516256**

(43) Veröffentlichungstag der Anmeldung: **12.11.86 Patentblatt 86/46**

(45) Bekanntmachung des Hinweises auf die Patenterteilung: **02.08.89 Patentblatt 89/31**

(84) Benannte Vertragsstaaten: **BE CH DE FR GB IT LI NL**

(56) Entgegenhaltungen: **DE-A-2 647 890**

(73) Patentinhaber: **BAYER AG, D-5090 Leverkusen 1 Bayerwerk (DE)**

(72) Erfinder: **Reiners, Jürgen, Dr., Carl- Rumpff-Strasse 57, D-5090 Leverkusen 1 (DE)**
Erfinder: **Winkel, Jens, Dr., Hahnenweg 6, D-5000 Koeln 80 (DE)**
Erfinder: **Klauke, Erich, Dr., Eichendorffweg 8, D-5068 Odenthal (DE)**
Erfinder: **Süling, Carlhans, Dr., Carl- Leverkus-Strasse 10, D-5068 Odenthal (DE)**
Erfinder: **Podszun, Wolfgang, Dr., Wolfskaul 4, D-5000 Koeln 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



LIBER, STOCKHOLM 1989

**Beschreibung**

Die Erfindung betrifft neue fluorhaltige Acrylsäure- und Methacrylsäureester, im folgenden (Meth)-Acrylsäureester genannt, und ihre Herstellung. Die neuen Verbindungen können als Monomere für die Anwendung im Dentalbereich eingesetzt werden.

Fluorhaltige Phenylcarbinol-acrylate wie 1,1,1,3,3,3-Hexafluor-2-Phenyl-2-acryloxy-propan sind aus Org. Coat. Plast. Chem. 42, 204 - 207, (1980) bekannt. Ähnlich aufgebaute (Meth)acrylsäureester, wie 1,3-Bis-(2-(meth)acryloxy-1,1,1,3,3,3-hexafluoropropyl-2)-5-perfluoralkyl-benzol und ihre Verwendung auf dem Dentalgebiet werden in der US-4 356 296 beschrieben. Durch die Trifluormethylgruppen werden die Carbinole acidifiziert und die daraus hergestellten Carbinolester zeichnen sich durch eine verminderte Hydrolysenbeständigkeit aus. Dadurch ist ihre Verwendbarkeit als Dentalmonomere eingeschränkt.

Weiterhin ist die Verwendung von 1,1,5-Trihydro-octafluoro-pentyl-methacrylat in Zahnfüllmassen in J. Dent. Res. 58, 1181 - 1186 (1979) beschrieben.

Monomere dieses Typs liefern Dentalmaterialien mit unzureichenden mechanischen Eigenschaften.

Es wurden neue (Meth)-Acrylsäureester der Formel

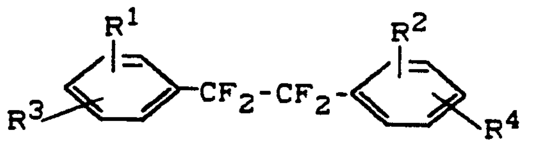

(I),

in der

$R^1$ und $R^2$ gleich oder verschieden sind und Wasserstoff, Chlor, Fluor oder einen $C_1$- bis $C_4$-Alkylrest bedeuten und

$R^3$ und $R^4$ gleich oder verschieden sind und für die Gruppe

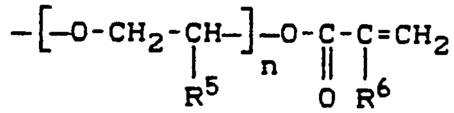

oder

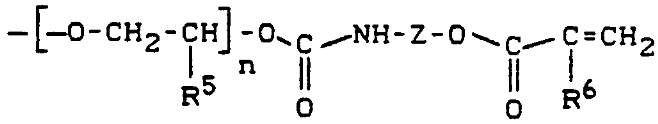

stehen, wobei

$R^5$ und $R^6$ gleich oder verschieden sind und ein Wasserstoffatom oder eine Methylgruppe bedeuten,

Z eine geradkettige oder verzweigte $C_2$- bis $C_8$-Alkylenkette bedeutet und

n Werte von 1 bis 4 bedeutet,

gefunden.

Die neuen (Meth)-Acrylsäureester sind farblos, schwerflüchtig und ergeben nach Polymerisation transparente Kunststoffe.

Sie lassen sich besonders gut in Abdichtungsmitteln, Klebstoffen und Dentalmaterialien, wie Zahnfüllmassen und Beschichtungsmitteln, verwenden. Die so erhaltenen Materialien zeichnen sich durch eine große Widerstandfähigkeit gegenüber physikalischer und chemischer Beanspruchung aus. Besonders hervorzuheben sind die günstigen Oberflächeneigenschaften und geringe Wasseraufnahme der mit den neuen (Meth)-Acrylsäureestern erhaltenen Polymerisate.

Im Rahmen der vorliegenden Erfindung können die Substituenten im allgemeinen folgende Bedeutung haben.

Ein $C_1$- bis $C_4$-Alkylrest bedeutet im allgemeinen ein geradkettiger oder verzweigter Kohlenwasserstoffrest. Beispielsweise seien Methyl, Ethyl, Propyl, iso- Propyl, Butyl und iso-Butyl genannt. Bevorzugt wird der Methylrest.

Ein $C_2$- bis $C_8$-Alkylenkette bedeutet im allgemeinen eine geradkettige oder verzweigte zweibindige Kohlenwasserstoffkette. Beispielsweise seien Ethylen, Propylen, iso-Propylen, Butylen, iso-Butylen, Pentylen, iso-Pentylen, Hexylen, iso-Hexylen, Heptylen, iso-Heptylen, Octylen und iso-Octylen genannt.

Bevorzugte (Meth)-Acrylsäureester sind Verbindungen der Formel

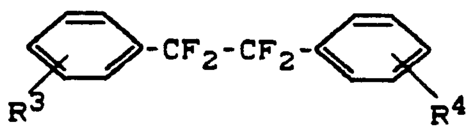 II

in der

$R^3$ und $R^4$ gleich oder verschieden sind und für die Gruppen

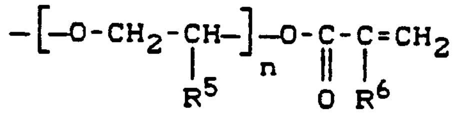 oder

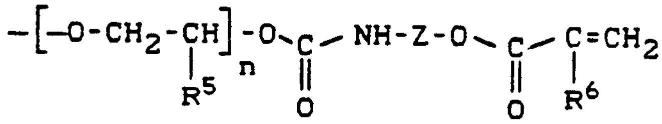

stehen, wobei

$R^5$ und $R^6$ gleich oder verschieden sind und ein Wasserstoffatom oder eine Methylgruppe bedeuten,

Z eine geradkettige oder verzweigte $C_2$- bis $C_8$-Alkylenkette bedeutet und

n Werte von 1 bis 4 bedeutet.

Die Substituenten $R^3$ und $R^4$ können bevorzugt in 3,3'- oder 3,4'- oder 4,4'-Stellung im 1,2-Diphenyltetrafluorethan stehen. Insbesondere bevorzugt werden Verbindungen mit $R^3$ und $R^4$ in 4,4'-Stellung.

Beispielsweise seien die folgenden (Meth)-Acrylsäureester genannt:

**Tabelle 1**

1 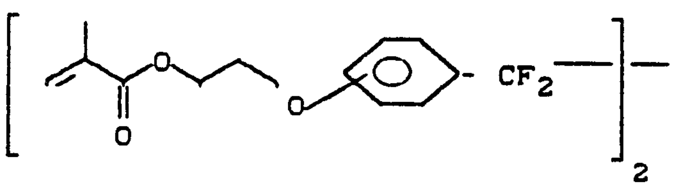

2 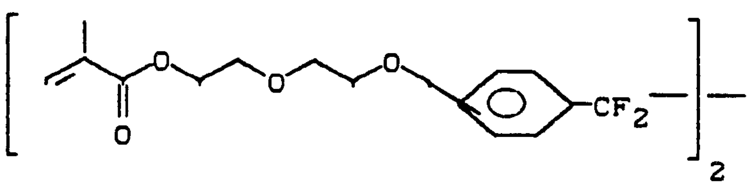

3 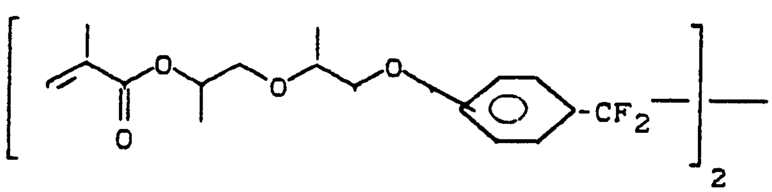

4 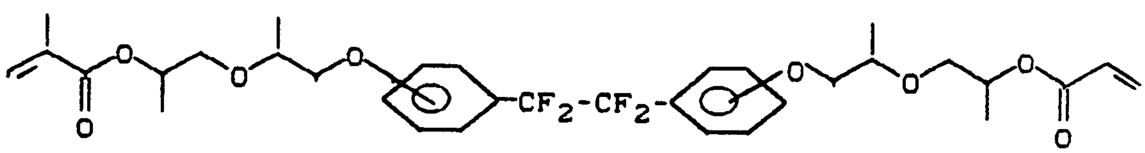

5 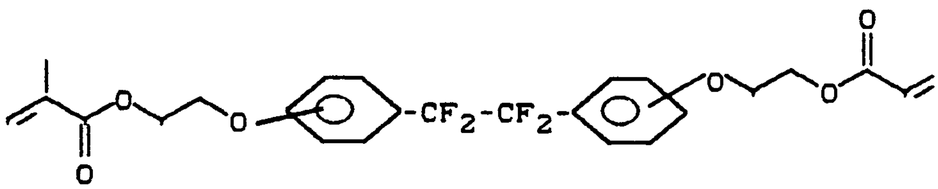

6 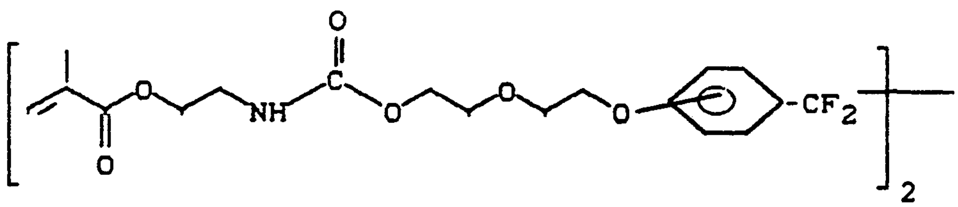

7 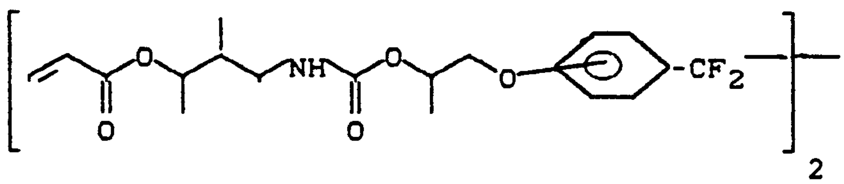

8 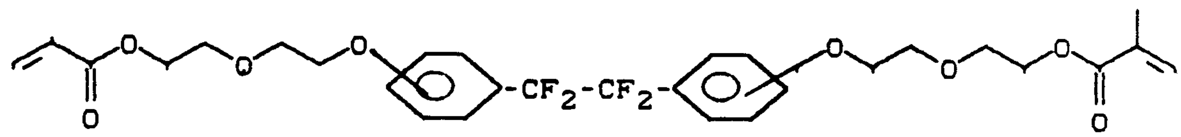

Es wurde auch ein Verfahren zur Herstellung der neuen (Meth)-Acryl-säureester gefunden. Das Herstellungsverfahren ist dadurch gekennzeichnet, daß man ein 1,2-Bis-(fluorphenyl)-1,1,2,2-tetrafluorethan der Formel

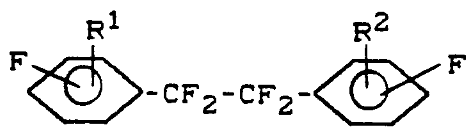

(III)

in der

$R^1$ und $R^2$ gleich oder verschieden sind und Wasserstoff, Chlor, Fluor oder einen $C_1$- bis $C_4$-Alkylrest bedeuten,

mit α,ω-Dihydroxy-verbindungen der Formel

$$HO-[CH_2-CHR^5-O-]-_nH \qquad (IV),$$

in der

n Werte von 1 bis 4 und

$R^5$ Wasserstoff oder Methyl bedeutet,

in Gegenwart einer starken Base verethert und das Reaktionsprodukt mit (Meth)acrylsäure und/oder seinen reaktiven Derivaten verestert oder mit Isocyanatoalkyl-(meth)-acrylaten umsetzt.

Verbindungen der Formel III sind beispielsweise gemäß L.M. Yagupolskii, V.I. Troitskaya, Zh. Obshch. Khim. 35. (9), 1620 (1965) bzw. J. Gen. Chem.. U.S.S.R. 35, 1616 bis 1623 (1965) zugänglich.

α,ω-Dihydroxyverbindungen können beispielsweise Ethylenglykol, Propylenglykol, Diethylenglykol oder Triethylenglykol sein.

Reaktive Derivate der (Meth)-acrylsäure sind beispielsweise die Säurehalogenide, bevorzugt die Chloride und Ester, bevorzugt Ester niederer Alkohole.

Die Oxyalkylierung der Verbindungen III mit den oben beschriebenen α,ω-Dihydroxyverbindungen wird in Gegenwart starker Basen im Temperaturbereich von 50 bis 180° C, bevorzugt 100 bis 160° C, durchgeführt.

Starke Basen können erfindungsgemäß einen $PK_B$-Wert von kleiner als 3, bevorzugt kleiner als 2, insbesondere bevorzugt kleiner als 0, haben. Beispielsweise seien Kalium-tert.-butylat, Natrium- und Kaliumisopropylat genannt.

Die Produkte werden beispielsweise isoliert, indem man die Reaktionsmischung auf Eiswasser gibt und die ausgefallenen Kristalle absaugt oder bei flüssigen Produkten mit einem wasserunlöslichen Lösungsmittel extrahiert.

Die durch die Oxyalkylierung erhaltenen Zwischenprodukte haben die allgemeine Formel

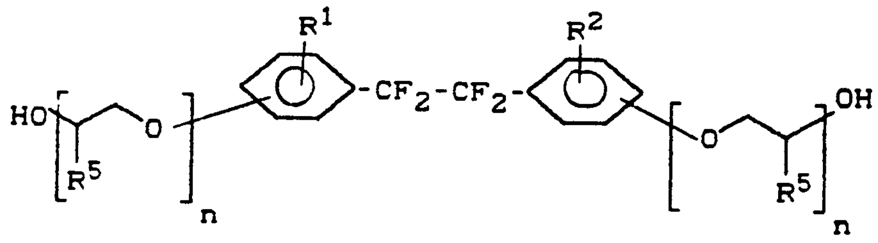

(V),

in der
$R^1$ und $R^2$ die oben erwähnte Bedeutung haben,
$R^5$ Wasserstoff oder eine Methylgruppe und
n Werte von 1 bis 4 bedeutet.

Die Anzahl der angelagerten Oxyalkylen-Einheiten (n) ist in weiten Grenzen variabel. Bevorzugt kann n die Werte 1 oder 2 annehmen.
Als besonders vorteilhaft erweisen sich Hydroxylverbindungen der Formel

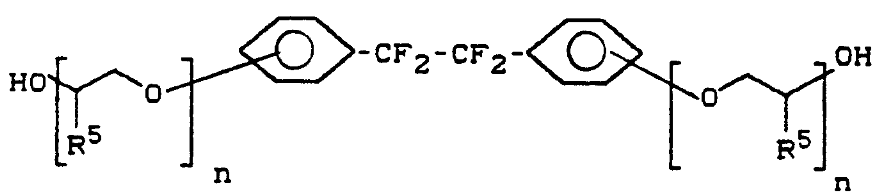

(VI),

in der
n Werte von 1 bis 4 annimmt und
$R^4$ ein Wasserstoffatom oder eine Methylgruppe bedeutet.

Die erfindungsgemäßen (Meth)Acrylsäureester (1) werden aus den Hydroxylverbindungen der Formel (V) durch Veresterung beziehungsweise durch Umsetzung mit Isocyanatoalkyl(meth)acrylaten erhalten.

Zur Veresterung können (Meth)-Acrylsäure, (Meth)acrylsäurechlorid, (Meth)-Acrylsäureanhydrid oder (Meth)Acrylsäureester niederer Alkohole eingesetzt werden. Die Veresterung erfolgt vorzugsweise mit (Meth)Acrylsäure in Anwesenheit eines sauren Katalysators, zum Beispiel p-Toluolsulfonsäure, Schwefelsäure oder Ionenaustauschern in der $H^{\oplus}$-Form in einem Lösungsmittel, das mit Wasser nicht mischbar ist, zum Beispiel Toluol, Chloroform, Xylol usw.

Die Veresterung kann beispielsweise wie folgt durchgeführt werden:

Die Hydroxylverbindung und ein Überschuß von (Meth)-Acrylsäure werden in einem Lösungsmittel suspendiert oder gelöst und mit dem sauren Katalysator sowie einem Polymerisations-Inhibitor versetzt. Das während der Veresterung gebildete Wasser wird durch azeotrope Destillation aus dem Gleichgewicht entfernt. Die Reaktion wird im allgemeinen im Temperaturbereich von 50°C bis etwa 120°C (Siedepunkt des azeotropen Gemisches etwa 100°C) durchgeführt.

Geeignete Polymerisations-Inhibitoren sind beispielsweise 2,6-Di-tert.-Butyl-4-methyl-phenol, Methylenblau und Hydrochinon in einer Menge von 0,01 bis 1 Gew.-%. Nach beendeter Veresterung wird nicht umgesetzte (Meth)-Acrylsäure durch Extraktion mit einer basischen wäßrigen Lösung entfernt. Der Inhibitor wird beispielsweise durch Zusatz von Adsorbentien abgetrennt. Die erfindungsgemäßen Reaktionsprodukte werden durch Abdestillieren der Lösungsmittel isoliert.

Zur Umsetzung der Hydroxylverbindungen gemäß Formel V mit Isocyanatoalkyl-(Meth)-Acrylaten eignen sich vorzugsweise Isocyanate der Formel:

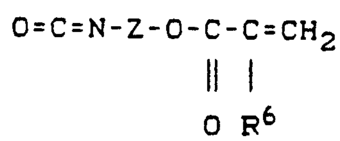

(VII)

wobei
Z eine geradkettige oder verzweigte $C_2$- bis $C_8$-Alkylenkette und
$R^6$ ein Wasserstoffatom oder eine Methylgruppe bedeuten. Geeignete Isocyanatoalkyl(meth)acrylate sind 2-Isocyanatoethylmethacrylat, 2-Isocyanatopropylmethacrylat und 1,2-Dimethyl-3-isocyanatopropylacrylat.

Die Umsetzung zum Urethan erfolgt vorzugsweise unter Wasserausschluß in einem inerten Lösungsmittel.

Beispiele für geeignete Lösungsmittel sind: Chloroform, Tetrahydrofuran, Dioxan, Methylenchlorid, Toluol und Acetonitril. Bevorzugte Lösungsmittel sind Chloroform, Tetrahydrofuran und Acetonitril.

Die Umsetzung wird im allgemeinen im Temperaturbereich von 20 bis 100°C, vorzugsweise 30 bis 70°C durchgeführt. Zur Beschleunigung der Umsetzung werden vorzugsweise zinnhaltige Katalysatoren wie Dibutylzinndilaurat oder Zinn(II)octoat verwendet. Andere geeignete Katalysatoren sind Verbindungen mit tert. Aminogruppen und Titanverbindungen. Im allgemeinen wird der Katalysator in einer Menge von 0,01 bis 2,5 Gew.-%, bevorzugt von 0,1 bis 1,5 Gew.-%, bezogen auf die Gesamtmenge der Reaktanden, eingesetzt.

Die Umsetzung zum Urethan wird im allgmeinen in Gegenwart von 0,01 bis 0,2 Gew.-% eines Polymerisations-Inhibitors, beispielsweise 2,6-Di-tert.-Butyl-4-methylphenol bei Normaldruck durchgeführt. Es ist aber auch möglich, das erfindungsgemäße Verfahren bei einem Unter- oder Überdruck durchzuführen.

Die Reaktion kann beispielsweise wie folgt durchgeführt werden:

Ein Isocyanatoalkyl(meth)acrylat (VII) und eine Hydroxylverbindung (V) werden in dem Lösungsmittel gelöst oder suspendiert und unter Rühren mit dem Katalysator versetzt.

Nach Zugabe des Polymerisations-Inhibitors wird die Reaktionslösung auf eine Temperatur zwischen 20 und 100°C erwärmt.

Der zeitliche Verlauf der Umsetzung kann beispielsweise durch Messung der IR-Spektren verfolgt werden. Nach vollständiger Umsetzung der Isocyanatgruppen werden die Reaktionsprodukte durch Entfernen des Lösungsmittels isoliert. Eine vorherige Reinigung mit Hilfe von Adsorbentien, beispielsweise Aktivkohle, Bleicherde, Kieselgel oder Aluminiumoxid ist möglich.

Für die Anwendung als Monomere für Zahnfüllmassen oder Beschichtungsmittel (Zahnlacke) im Dentalbereich können die erfindungsgemäßen (Meth)-Acrylsäureester der Formel I mit an sich bekannten Monomeren gemischt werden, um beispielsweise die Viskosität dem Verwendungszweck anzupassen. Viskositäten im Bereich von 60 bis 10000 mPas sind dabei bevorzugt. Dies ist dadurch erreichbar, daß man den erfindungsgemäßen Monomeren gegebenenfalls ein Comonomer niedrigerer Viskosität als Reaktivverdünner zumischt. Die erfindungsgemäßen Verbindungen werden in der Mischung mit Comonomeren mit einem Anteil von ca. 30 bis ca. 90 Gew.-% eingesetzt, wobei ein Anteil von 50 bis 90 Gew.-% besonders bevorzugt ist.

Im Rahmen der vorliegenden Erfindung ist es ebenfalls möglich, Mischungen verschiedener erfindungsgemäßer (Meth)-Acrylsäureester einzusetzen.

Es ist auch möglich, Monomermischungen einzusetzen, die mehrere Comonomere als Reaktivverdünner enthalten.

Beispielsweise seien die folgenden Comonomere genannt:

Triethylenglykoldimethacrylat, Tetraethylenglykoldimethacrylat, 1,12-Dodecandioldimethacrylat, 1,6-Hexandioldimethacrylat, Diethylenglykoldimethacrylat, (Meth)acrylsäureester ethoxylierter oder propoxylierter hydroxylgruppenhaltiger Tricyclo[5.2.1.$0^{2,6}$]decan-Derivate (vgl. DE-OS-2 931 925 und 2 931 926). Insbesondere werden Comonomere bevorzugt, die bei 13 mbar einen Siedepunkt über 100°C besitzen.

Die erfindungsgemäßen (Meth)-Acrylsäureester lassen sich, gegebenenfalls in Mischung mit dem genannten Monomeren, mit an sich bekannten Methoden aushärten (G. M. Brauer, H. Argentar, Am. Chem. Soc., Symp. Ser. 212, S 359 - 371 (1983)). Für die sogenannte Redoxpolymerisation ist ein System aus einer peroxidischen Verbindung und einem Reduktionsmittel, beispielsweise auf Basis tertiärer aromatischer Amine, geeignet, wobei die peroxidhaltige und aminhaltige Monomermischung bis zur Verwendung getrennt gelagert werden müssen, um eine vorzeitige Polymerisation zu verhindern.

Beispiele für geeignete Peroxide sind:

Dibenzoylperoxid, Dilauroylperoxid und Di-Chlorbenzoylperoxid.

Als tertiäre aromatische Amine seien beispielsweise N,N-Dimethyl-p-toluidin, Bis-(2-hydroxyethyl)-p-toluidin, Bis(2-hydroxyethyl)-3,5-dimethylanilin und das in der DE-PS-2 759 239 beschriebene N-Methyl-N-(2-Methylcarbamoyloxypropyl)-3,5-dimethylanilin genannt.

Die Konzentration des Peroxids bzw. des Amins werden vorteilhaft so gewählt, daß sie 0,1 bis 5 Gew.-%, bevorzugt 0,5 bis 3 Gew.-%, bezogen auf die Monomermischung betragen.

Die erfindungsgemäßen Monomeren können auch durch Bestrahlung mit UV-Licht oder sichtbarem Licht (beispielsweise im Wellenlängenbereich von 230 bis 650 nm) zur Polymerisation gebracht werden.

Es handelt sich dabei um ein Einkomponentensystem. Der Vorteil gegenüber redoxhärtenden Zweikomponentensystemen besteht darin, daß die Aushärtung der Monomermischung nicht durch Inhomogenitäten beeinträchtigt wird, die beim Redoxsystem durch unzureichende Durchmischung der beiden Komponenten verursacht werden können.

Als Initiatoren für die photoinitiierte Polymerisation eignen sich beispielsweise Benzil, Benzildimethylketal, Benzoinmonoalkylether, Benzophenon, p-Methoxybenzophenon, Fluorenon, Thioxanthon, Phenanthrenchinon und 2,3-Bornandion (Campherchinon), gegebenenfalls in Gegenwart von synergistisch wirkenden Photoaktivatoren, wie N,N-Dimethylaminoethylmethacrylat, Triethanolamin, 4-N,N-Dimethylaminobenzolsulfonsäurebisallylamid. Die Durchführung des Photopolymerisationsverfahrens ist beispielsweise in der DE-PS-3 135 115 beschrieben.

Neben den oben beschriebenen Initiatoren können den erfindungsgemäßen (Meth)-Acrylsäureestern an sich für diesen Einsatzzweck bekannte Lichtschutzmittel und Polymerisations-Inhibitoren zugesetzt werden.

Das Lichtschutzmittel und der Polymerisations-Inhibitor werden im allgemeinen in einer Menge von 0,01 bis 0,50 Gew.-Teilen, bezogen auf 100 Gew.-Teile der Monomermischung eingesetzt. Die Monomermischungen können ohne Zusatz von Füllstoffen als Beschichtungsmittel (Zahnlacke) eingesetzt werden.

Bei der Verwendung als Zahnfüllmassen setzt man den erhaltenen Monomermischungen im allgemeinen Füllstoffe zu. Um einen hohen Füllgrad erreichen zu können, sind Monomermischungen, die eine Viskosität im Bereich von 60 bis 10000 mPas besitzen, besonders vorteilhaft. Den die erfindungsgemäßen Verbindungen der Formel I enthaltenden Monomermischungen können vorzugsweise anorganische Füllstoffe zugemischt werden. Beispielsweise seien Bergkristall, Quarzit, Kristobalit, Quarzglas, hochdisperse Kieselsäure, Aluminiumoxid und Glaskeramiken, beispielsweise Lanthan und Zirkon enthaltende Glaskeramiken (DE-OS-2 347 591) genannt.

Die anorganischen Füllstoffe werden zur Verbesserung des Verbundes zur Polymermatrix des Polymethacrylats vorzugsweise mit einem Haftvermittler vorbehandelt. Die Haftvermittlung kann beispielsweise durch eine Behandlung mit Organosiliciumverbindungen erreicht werden (Plueddemann, Progress in Organic coatings, 11, 297 bis 308 (1983)). Bevorzugt wird 3-Methacryloyloxypropyl-trimethoxysilan eingesetzt.

Die Füllstoffe für die erfindungsgemäßen Zahnfüllmassen weisen im allgemeinen einen mittleren Teilchendurchmesser von 0,01 bis 100 µm, vorzugsweise von 0,05 bis 50 µm auf, besonders bevorzugt 0,05 bis 5 µm. Es kann auch vorteilhaft sein, mehrere Füllstoffe nebeneinander einzusetzen, die einen voneinander verschiedenen Teilchendurchmesser besitzen.

Der Anteil der erfindungsgemäßen (Meth)-Acrylsäureester in den Füllmassen beträgt im allgemeinen 5 bis 85 Gew.-%, bezogen auf die Füllmasse.

Der Füllstoffanteil in den Zahnfüllmassen beträgt im allgemeinen 5 bis 85 Gew.-%, vorzugsweise 50 bis 80 Gew.-%.

Für die Herstellung der Zahnfüllmassen werden die Komponenten unter Verwendung von an sich bekannten Knetmaschinen verarbeitet.

## Herstellungsbeispiele

### Beispiel 1

A) Herstellung von 1,2-Bis[4-(2-Hydroxyethoxy)phenyl]-1,1,2,2-tetrafluorethan

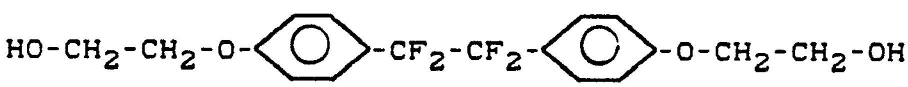

700 ml Ethylenglykol und 65 g 1,2-Bis(4-Fluorphenyl)-1,1,2,2-tetrafluorethan* werden in einem Rührkolben auf 110° C erhitzt. Man gibt 57 g Kalium-tert.-butylat portionsweise zu und rührt anschließend 7 Stunden bei 150° C. Der Ansatz wird abgekühlt, auf 4 l Eiswasser gegossen und mit einem Homogenisator (Ultraturax) durchmischt. Die ausgefallenen Kristalle werden abgesaugt, nochmal mit 2 l Wasser verrührt, wieder abgesaugt, getrocknet und aus Toluol umkristallisiert.

Ausbeute: 39 g
Schmelzpunkt: 124 - 127° C
Das $^1$-H-NMR-Spektrum beweist die oben angegebene Struktur.

B) Herstellung von 1,2-Bis[4-(2-Hydroxyethoxyethoxy)-phenyl]-1,1,2,2-tetrafluorethan

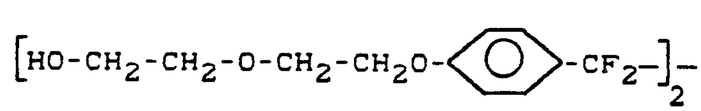

700 ml Diethylenglykol und 65 g 1,2-Bis(4-Fluorphenyl)-1,1,2,2-tetrafluorethan* werden in einem Rührkolben auf 130° C erhitzt und 57 g Kaliumtert.-butylat portionsweise zugegeben. Anschließend wird auf 150° C erhitzt und 2 Stunden bei dieser Temperatur gerührt. Der Ansatz wird abgekühlt, auf 4 l Eiswasser gegeben und mit einem Homogenisator (Ultraturax) durchmischt, bis Kristalle ausfallen. Der Niederschlag wird abgesaugt, nochmal mit 3 l Wasser verrührt, mit Salzsäure sauergestellt, wieder abgesaugt, getrocknet und aus Toluol umkristallisiert.

Ausbeute: 70 g
Schmelzpunkt: 94 - 95° C
Das integrierte $^1$H-NMR-Spektrum betätigt die oben angegebene Struktur
* Chem. Abstr. Reg. NO [4100-99-6]

**Beispiel 2** (4,4'-Isomer der Verbindung 1 aus Tabelle 1)

Herstellung von 1,2-Bis-[4-(2-Methacryloyloxy-ethoxy)-phenyl]-1,1,-2,2-tetrafluorethan

37,4 g (0,1 mol) 1,2-Bis-[4-(2-Hydroxyethoxy)phenyl]-1,1,2,2,-tetrafluorethan
25,8 g (0,3 mol) Methacrylsäure
1 g p-Toluolsulfonsäure und
0,3 g Methylenblau

werden in 100 ml Toluol suspendiert. Bei 110°c wird durch azeotrope Destillation das Wasser ausgekreist, wobei durch die Suspension Luft geleitet wurde. Nach beendeter Wasserabscheidung wird mit Bleicherde ausgerührt, abgesaugt und das Filtrat mit Natriumhydrogencarbonat-Lösung extrahiert. Die organische Phase wird mit Zellstoffmehl verrührt und abgesaugt. Anschließend wird mit Natriumchlorid-Lösung neutral gewaschen. Die organische Phase wird über Natriumsulfat getrocknet und im Vakuum vom Lösungsmittel befreit.

Ausbeute: 39,3 g (77 %)
Schmelzpunkt: 84 bis 85°C
GC-Analyse: 97 %

**Beispiel 3** (4,4'-Isomer der Verbindung 2 aus Tabelle 1)
Herstellung von 1,2-Bis-[4-(2-Methacryloyloxy-ethoxy-ethoxy)phenyl]1,1,2,2-tetrafluorethan

46,2 g (0,1 mol) 1,2-Bis-[4-(2-Hydroxyethoxyethoxy)-phenyl]1,1,2,2-tetrafluorethan
25,8 g (0,3 mol) Methacrylsäure
1 g p-Toluolsulfonsäure und
0,3 g Methylenblau werden in 150 ml Toluol suspendiert.

Unter Einleiten von Luft wird während 24 Stunden das Reaktionswasser kontinuierlich ausgekreist. Nach beendeter Reaktion wird mit Bleicherde ausgerührt, abgesaugt und das Filtrat mit Natriumhydrogencarbonat-Lösung extrahiert. Die organische Phase wird mit Zellstoffmehl verrührt und abgesaugt. Anschließend wird mit Natriumchlorid-Lösung neutral gewaschen. Die Toluolphase wird über Natriumsulfat getrocknet und im Vakuum vom Lösungsmittel befreit.

Ausbeute: 53,5 g (89,5 %)
Schmelzpunkt: 64 bis 66°C
$^1$H-NMR ($CDCl_3$) [ppm]: 1,95 ($-CH_3$, 6H), 4,5 - 3,7 ($-CH_2-$, 16H), 5,5 - 5,65 und 6,2 - 6,05 ($=CH_2$, 4H), 6,75 - 7,5 (1,4-disubstituierter Phenylrest, 8H)

**Beispiel 4** (4,4'-Isomer der Verbindung 6 aus Tabelle 1)

Herstellung von 1,2-Bis[4-(2-Methacryloyloxyethyl-carbamoyloxy-ethoxyethoxy)phenyl]-1,1,2,2-tetrafluorethan

4,62 g (10 mmol) 1,2-Bis-[4-(2-Hydroxyethoxyethoxy)-phenyl]-1,1,2,2-tetrafluorethan werden in 30 ml Chloroform gelöst.

0,03 g Zinn(II)octoat und 0,003 g 2,6-Di-tert.-Butyl-4-methylphenol werden zugegeben. Langsam werden 3,1 g (20 mmol) 2-Isocyanatoethylmethacrylat bei Raumtemperatur zugetropft. Nach beendeter Zugabe des Isocyanats wird die Reaktionsmischung bei 50°C gerührt, bis die IR-Absorption der NCQ-Bande bei 2200 $cm^{-1}$ verschwunden ist (etwa 5 Stunden Reaktionszeit). Das Produkt wird durch Entfernen des Lösungsmittels im Vakuum isoliert. Das Urethan ist eine farblose, viskose Flüssigkeit, die beim Abkühlen unter 10°C nach mehreren Tagen kristallin wird.

Ausbeute: 7,6 g (98,4 %)
Die erwartete Struktur wurde durch das $^1$H-NMR-Spektrum bestätigt.

**Beispiel 5** (4,4'-Isomer der Verbindung 8 aus Tabelle 1)

Herstellung von 1,2-[4-(2-Methacryloyloxyethoxyethoxy)-4-(2-acryloyloxy-ethoxyethoxy)]-diphenyl-1,1,2,2-tetrafluorethan

46,2 g (0,1 mol) 1,2-Bis-[4-2-Hydroxyethoxyethoxy)-phenyl]-1,1,2,2-tetrafluorethan
12,9 g (0,15 mol) Methacrylsäure
10,8 g (0,15 mol) Acrylsäure
1 g p-Toluolsulfonsäure und
0,3 g Methylenblau

werden in 250 ml Toluol suspendiert und 24 Stunden unter Rückfluß erhitzt.

Die Reaktion und die Aufarbeitung werden analog zu Beispiel 3 geführt. Das Monomer ist nach vollständiger Entfernung des Lösungsmittels eine farblose, viskose Flüssigkeit.
Ausbeute: 51,7 g

**Anwendungsbeispiele:**

**Beispiel 6**

Herstellung von Beschichtungs-Lösungen

a) redoxhärtendes System

In einer Lösung aus dem in Beispiel 5 genannten Monomer (80 Gew.-Teile) und Triethylenglykoldimethacrylat (20 Gew.-Teile) werden 2,00 Gew.-% Di-Benzoylperoxid und 0,04 Gew.-% 2,6-Di-tert.-Butyl-4-methylphenol gelöst.
In einer zweiten, kein Peroxid enthaltenden, sonst aber gleich zusammengesetzten Mischung werden 2,2 Gew.-% N-Methyl-N-(2-Methylcarbamoyloxy-propyl)-3,5-dimethylanilin gelöst.
Eine Mischung aus gleichen Teilen der beiden zuvor beschriebenen Lösungen härtet in 2 bis 3 Minuten aus.

b) lichthärtendes System

In einer Monomermischung aus 80 Gew.-Teilen Monomer aus Beispiel 5 und 20 Gew.-Teilen Triethylenglykoldimethacrylat werden 0,5 Gew.-% 4-N,N-Dimethylaminobenzol-sulfonsäure-bis-allylamid, 0,125 Gew.-% Benzildimethylketal, 0,2 Gew.-% Bicyclo[2,2,1]-1,7,7-trimethyl-heptan-2,3-dion (2,3-Bornandion) und 0,04 Gew.-% 2,6-Ditert.-Butyl-4-methylphenol gelöst.
Beim Belichten mit einer Dentallampe härtet die Flüssigkeit aus (Belichtungszeit 40 Sekunden).

**Beispiel 7**

Beispiel 6 wurde unter Verwendung des Monomers aus Beispiel 4 wiederholt.
Die ausgehärteten Beschichtungslösungen aus Beispielen 6 und 7 sind transparent und weisen eine hohe Härte auf.

**Beispiel 8**

Herstellung einer redoxhärtenden Dentalmasse

Aminpaste: In einer Monomer-Mischung von 80 Gewichtsteilen der erfindungsgemäßen Verbindung aus Beispiel 5 und 20 Gewichtsteilen Triethylenglykoldimethacrylat werden 2,2 Gew.-% N-Methyl-N-(2-Methylcarbamoyloxypropyl)-3,5-dimethylanilin und 0,04 Gew.-% des Polymerisations-Inhibitors aus Beispiel 6 gelöst. 5 g dieser Lösung werden mit 15 g einer handelsüblichen Glaskeramik mit einem mittleren Teilchendurchmesser von 4 µm, die mit 3-Methacryloyloxypropyl-trimethoxysilan silanisiert wurde, zu einer Paste verarbeitet.

Peroxidpaste: In einer Mischung von 80 Gewichtsteilen der erfindungsgemäßen Verbindung aus Beispiel 5 und 20 Gewichtsteilen Triethylenglykoldimethacrylat werden 2,0 Gew.-% Dibenzoylperoxid gelöst. 5 g dieser Lösung werden mit 15 g einer handelsüblichen Glaskeramik mit einem mittleren Teilchendurchmesser von 4 µm, die mit 3-Methacryloyloxypropyl-trimethoxysilan silanisiert wurde, zu einer Paste verarbeitet.

Eine Mischung von gleichen Teilen Aminpaste und Peroxidpaste härtet innerhalb von 2 bis 3 Minuten aus.

**Beispiel 9**

Herstellung eines lichthärtenden Zahnfüllungsmaterials

In einer Mischung aus 80 Gewichtsteilen Monomer aus Beispiel 5 und 20 Gewichtsteilen Triethylenglykoldimethacrylat werden 0,2 Gew.-% 2,3-Bornandion, 0,125 Gew.-% Benzildimethylketal. 0,5 Gew.-% 4-N,N-Dimethylaminobenzolsulfonsäure-bis-allylamid und 0,04 Gew.-% 2,6-Ditert.-Butyl-4-Methylphenol gelöst.

5 g dieser Lösung werden mit 15 g des in Beispiel 8 beschriebenen Füllstoffs zu einer Paste verarbeitet (75 % Füllstoffgehalt).

Die Aushärtung erfolgt durch Belichtung mit einer Dentallampe. Bei einer Belichtungszeit von 40 Sekunden beträgt die Aushärtungstiefe 6,1 mm.

**Beispiel 10**

Beispiel 9 wurde unter Verwendung von Verbindung aus Beispiel 4 wiederholt.

**Beispiel 11**

Messung von Festkörperoberflächenspannungen

An dem ausgehärteten Beschichtungsmittel aus dem Beispiel 6 wurden Messungen der Oberflächenspannung durchgeführt. Mittels eines Video- Systems wurde das dynamische Benetzungsverhalten von Flüssigkeiten auf den Festkörperoberflächen bestimmt. Die Oberflächenspannungen wurden aus den Anfangsrandwinkeln von 5 Prüfflüssigkeiten berechnet. Die Ergebnisse sind in der Tabelle 2 zusammengestellt.

**Tabelle 2**

Messung der Festkörperoberflächenspannungen

| Monomer | Verhältnis Monomer zu TEGDMA[A)] | Gesamt [mN/m] | unpolarer Anteil [mN/m] | polarer Anteil [mN/m] | polarer Anteil [%] |
|---|---|---|---|---|---|
| Verbindung 8 | 80 : 20 | 37,3 | 31,3 | 6,0 | 16,1 |
| Vergleich [B)] | 70 : 30 | 42,2 | 28,7 | 13,5 | 32,0 |

[A)] TEGDMA = Triethylenglykoldimethacrylat
[B)] Bisphenol A-diglycidyl-dimethacrylat (Bis-GMA) /TEGDMA

**Patentansprüche**

1. (Meth)-Acrylsäureester der Formel

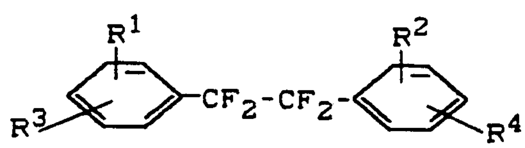

in der

$R^1$ und $R^2$ gleich oder verschieden sind und Wasserstoff, Chlor, Fluor oder einen $C_1$- bis $C_4$-Alkylrest bedeuten und

$R^3$ und $R^4$ gleich oder verschieden sind und für die Gruppe

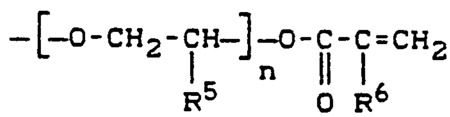

oder

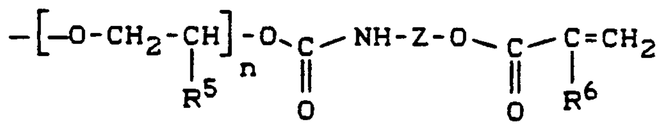

stehen, wobei

$R^5$ und $R^6$ gleich oder verschieden sind und ein Wasserstoffatom oder eine Methylgruppe bedeuten,

Z eine geradkettige oder verzweigte $C_2$- bis $C_8$-Alkylenkette bedeutet und

n Werte von 1 bis 4 bedeutet.

2. (Meth)-Acrylsäureester nach Anspruch 1 der Formel

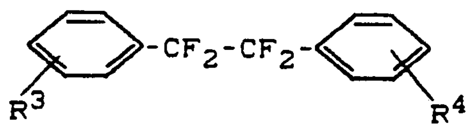

in der

$R^3$ und $R^4$ gleich oder verschieden sind und für die Gruppen

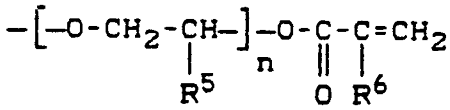

oder

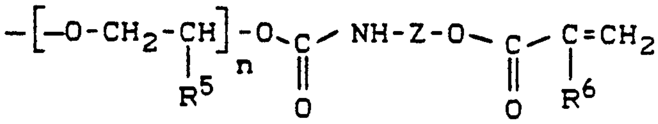

stehen, wobei

$R^5$ und $R^6$ gleich oder verschieden sind und ein Wasserstoffatom oder eine Methylgruppe bedeuten,

Z eine geradkettige oder verzweigte $C_2$- bis $C_8$-Alkylenkette bedeutet und

n Werte von 1 bis 4 bedeutet.

3. Verfahren zur Herstellung von (Meth)-Acrylsäureestern dadurch gekennzeichnet, daß man ein 1,2-Bis-(fluorphenyl)-1,1,2,2-tetrafluorethan der Formel

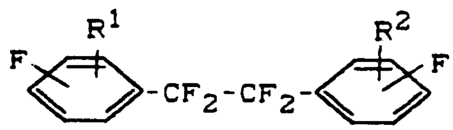

in der

$R^1$ und $R^2$ gleich oder verschieden sind und Wasserstoff, Chlor, Fluor oder einen $C_1$- bis $C_4$-Alkylrest bedeuten,

mit α,ω-Dihydroxy-Verbindungen der Formel

$HO-[-CH_2-CHR^5-O-]-_n H$

in der

$R^5$ Wasserstoff oder Methyl bedeutet und

n Werte von 1 bis 4 bedeutet,

verethert und das Reaktionsprodukt mit (Meth)-Acrylsäure und/oder seinen reaktiven Derivaten verestert oder mit Isocyanatoalkyl(meth)-acrylaten umsetzt.

4. Polymerisat aus (Meth)-Acrylsäureestern der Formel

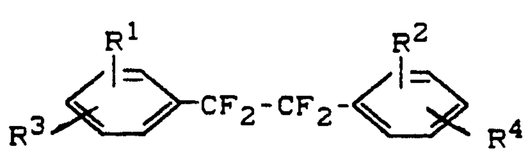

in der $R^1$ und $R^2$ gleich oder verschieden sind und Wasserstoff, Chlor, Fluor oder einen $C_1$- bis $C_4$-Alkylrest bedeuten und $R^3$ und $R^4$ gleich oder verschieden sind und für die Gruppe

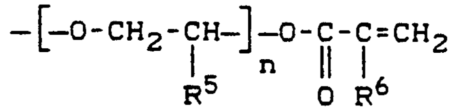

oder

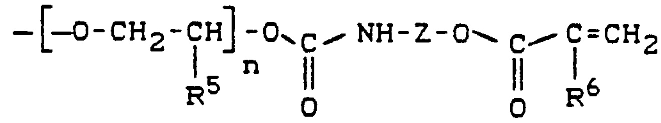

stehen, wobei $R^5$ und $R^6$ gleich oder verschieden sind, Wasserstoff oder Methyl bedeuten, Z eine geradkettige oder verzweigte $C_2$- bis $C_8$- Alkylenkette bedeutet und n Werte von 1 bis 4 bedeutet.

5. Verwendung von (Meth)-Acrylsäureestern nach Anspruch 1 im Dentalbereich.

6. Verwendung nach Anspruch 5, dadurch gekennzeichnet, daß die (Meth)-Acrylsäureester in Zahnfüllmassen eingesetzt werden.

7. Verwendung nach Anspruch 5, dadurch gekennzeichnet, daß die (Meth)-Acrylsäureester in Beschichtungsmitteln für Zähne eingesetzt werden.

8. Zahnfüllmassen, dadurch gekennzeichnet, daß sie (Meth)-Acrylsäureester der Formel

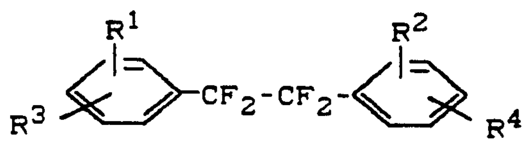

in der $R^1$ und $R^2$ gleich oder verschieden sind und Wasserstoff, Chlor, Fluor oder einen $C_1$- bis $C_4$-Alkylrest bedeuten und $R^3$ und $R^4$ gleich oder verschieden sind und für die Gruppe

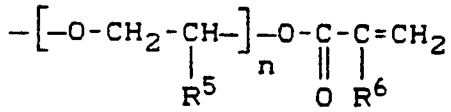

oder

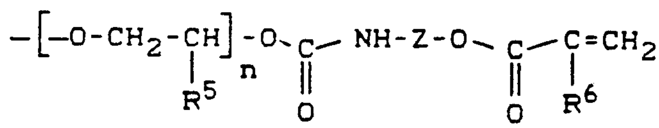

stehen, wobei $R^5$ und $R^6$ gleich oder verschieden sind, Wasserstoff oder Methyl bedeuten, Z eine geradkettige oder verzweigte $C_2$- bis $C_8$- Alkylenkette bedeutet und n Werte von 1 bis 4 bedeutet, enthalten.

## Claims

1. (Meth)-acrylic acid esters of the formula

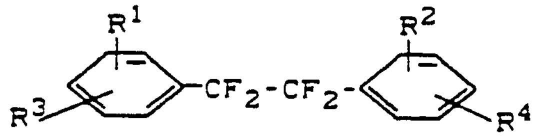

in which
$R^1$ and $R^2$ are identical or different and denote hydrogen, chlorine, fluorine or a $C_1$- to $C_4$-alkyl radical and
$R^3$ and $R^4$ are identical or different and represent the group

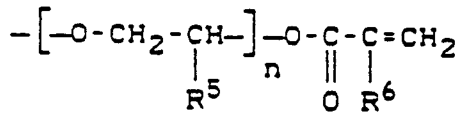

or

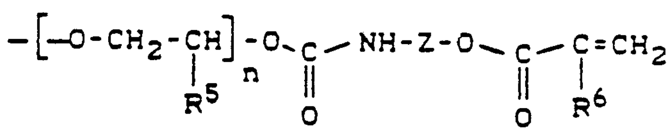

wherein
$R^5$ and $R^6$ are identical or different and denote a hydrogen atom or a methyl group,
Z denotes a straight-chain or branched $C_2$- to $C_8$-alkylene chain and
n denotes values from 1 to 4.

2. (Meth)-acrylic acid esters according to Claim 1, of the formula

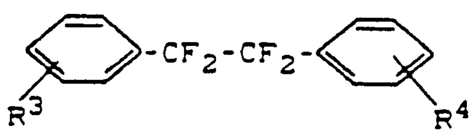

in which
$R^3$ and $R^4$ are identical or different and represent the groups

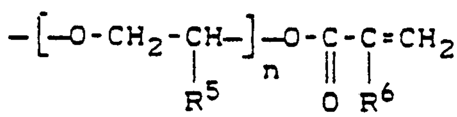

or

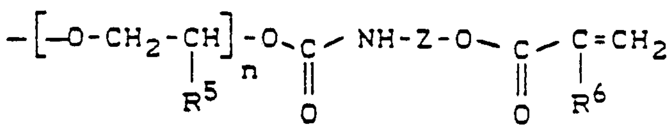

wherein
$R^5$ and $R^6$ are identical or different and denote a hydrogen atom or a methyl group,
Z denotes a straight-chain or branched $C_2$- to $C_8$-alkylene chain and
n denotes values from 1 to 4.

3. Process for the preparation of (meth)-acrylic acid esters, characterized in that a 1,2-bis-(fluorophenyl)-1,1,2,2-tetrafluoroethane of the formula

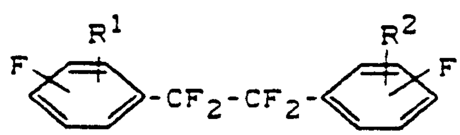

in which
$R^1$ and $R^2$ are identical or different and denote hydrogen, chlorine, fluorine or a $C_1$- to $C_4$-alkyl radical,
is etherified with α,ω-dihydroxy compounds of the formula

HO-[-$CH_2$-$CHR^5$-O-]-$_n$ H

in which

$R^5$ denotes hydrogen or methyl and

n denotes values from 1 to 4,

and the reaction product is esterified with (meth)-acrylic acid and/or its reactive derivatives or reacted with isocyanatoalkyl(meth)-acrylates.

4. Polymer of (meth)-acrylic acid esters of the formula

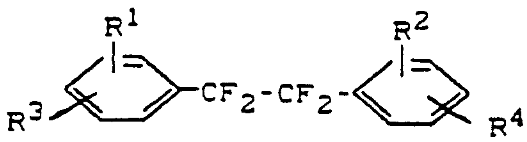

in which

$R^1$ and $R^2$ are identical or different and denote hydrogen, chlorine, fluorine or a $C_1$- to $C_4$-alkyl radical and

$R^3$ and $R^4$ are identical or different and represent the group

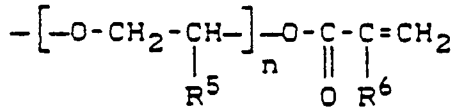

or

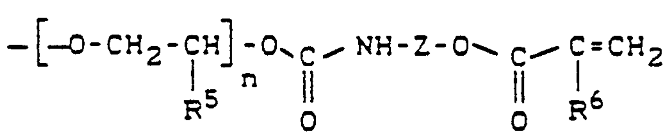

wherein

$R^5$ and $R^6$ are identical or different and denote hydrogen or methyl,

Z denotes a straight-chain or branched $C_2$- to $C_8$-alkylene chain and

n denotes values from 1 to 4.

5. Use of (meth)-acrylic acid esters according to Claim 1 in the dental field.

6. Use according to Claim 5, characterized in that the (meth)-acrylic acid esters are employed in dental filling compositions.

7. Use according to Claim 5, characterized in that the (meth)-acrylic acid esters are employed in coating agents for teeth.

8. Dental filling compositions, characterized in that they contain (meth)-acrylic acid esters of the formula

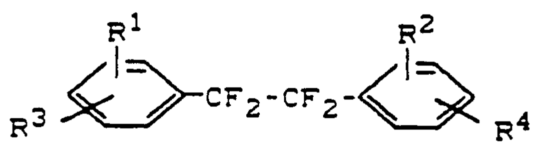

in which

$R^1$ and $R^2$ are identical or different and denote hydrogen, chlorine, fluorine or a $C_1$- to $C_4$-alkyl radical and

$R^3$ and $R^4$ are identical or different and represent the group

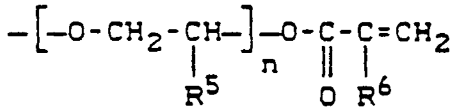

or

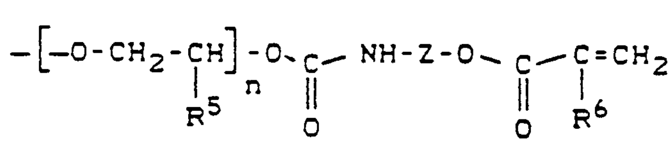

wherein

$R^5$ and $R^6$ are identical or different and denote hydrogen or methyl,

Z denotes a straight-chain or branched $C_2$- to $C_8$-alkylene chain and

n denotes values from 1 to 4.

## Revendications

1. Ester d'acide (méth)acrylique de formule

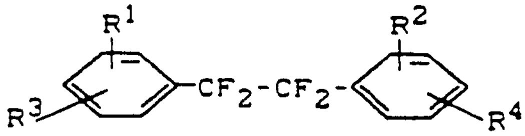

dans laquelle
$R^1$ et $R^2$ sont identiques ou différents et représentent l'hydrogène, le chlore, le fluor ou un reste alkyle en $C_1$ à $C_4$ et
$R^3$ et $R^4$ sont identiques ou différents et représentent le groupe

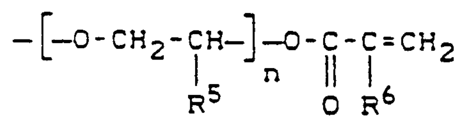

ou

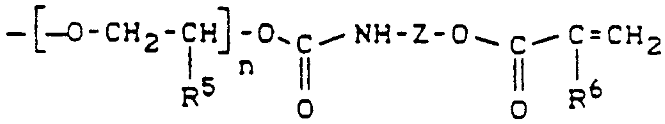

où
$R^5$ et $R^6$ sont identiques ou différents et représentent un atome d'hydrogène ou un groupe méthyle,
Z est une chaîne alkylénique droite ou ramifiée en $C_2$ à $C_8$ et
n a des valeurs de 1 à 4.

2. Ester d'acide (méth)acrylique suivant la revendication 1, de formule

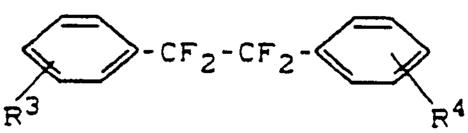

dans laquelle
$R^3$ et $R^4$ sont identiques ou différents et représentent les groupes

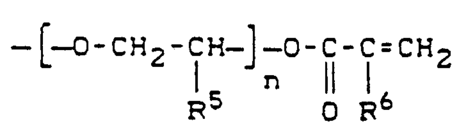

ou

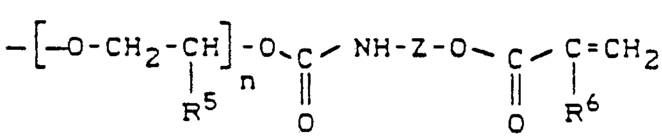

où
$R^5$ et $R^6$ sont identiques ou différents et désignent un atome d'hydrogène ou un groupe méthyle,
Z est une chaîne alkylénique droite ou ramifiée en $C_2$ à $C_8$ et
n a des valeurs de 1 à 4.

3. Procédé de production d'esters d'acide (méth)acrylique, caractérisé en ce qu'on éthérifie le 1,2-bis-(fluorophényl)-1,1,2,2-tétrafluoréthane de formule

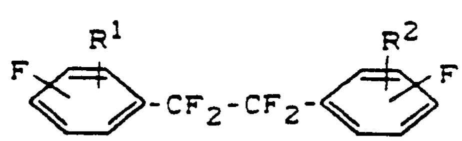

dans laquelle
$R^1$ et $R^2$ sont identiques ou différents et désignent l'hydrogène, le chlore, le fluor ou un reste alkyle en $C_1$ à $C_4$,
avec des composés α,ω-dihydroxyliques de formule

$HO\text{-}[\text{-}CH_2\text{-}CHR^5\text{-}O\text{-}]\text{-}_n H$

dans laquelle

$R^5$ désigne l'hydrogène ou le groupe méthyle et

n a des valeurs de 1 à 4,

et on estérifie le produit de réaction avec l'acide (méth)acrylique et/ou ses dérivés réactifs ou on les fait réagir avec des (méth)acrylates d'isocyanato-alkyle.

4. Polymérisat d'esters d'acide (méth)acrylique de formule

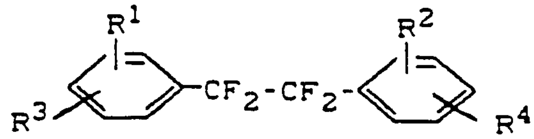

dans laquelle

$R^1$ et $R^2$ sont identiques ou différents et représentent l'hydrogène, le chlore, le fluor ou un reste alkyle en $C_1$ à $C_4$ et

$R^3$ et $R^4$ sont identiques ou différents et représentent le groupe

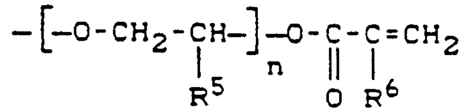

ou

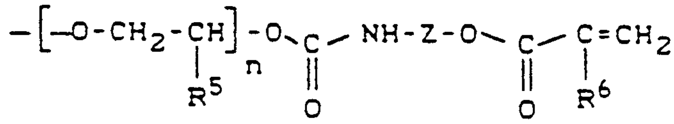

où

$R^5$ et $R^6$ sont identiques ou différents et représentent l'hydrogène ou le groupe méthyle,

Z est une chaîne alkylénique droite ou ramifiée en $C_2$ à $C_8$ et

n a des valeurs de 1 à 4.

5. Utilisation d'esters d'acide (méth)acrylique suivant la revendication 1 dans le domaine de l'art dentaire.

6. Utilisation suivant la revendication 5, caractérisée en ce que les esters d'acide (méth)acrylique sont utilisés dans des compositions d'obturation dentaire.

7. Utilisation suivant la revendication 5, caractérisée en ce que les esters d'acide (méth)acrylique sont utilisés dans des compositions dentaires de revêtement.

8. Compositions pour obturation dentaire, caractérisées en ce qu'elle contiennent des esters d'acide (méth)acrylique de formule

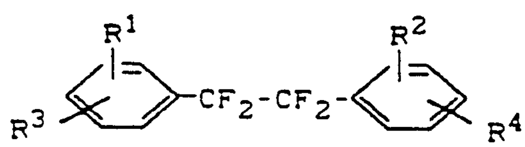

dans laquelle

$R^1$ et $R^2$ sont identiques ou différents et représentent l'hydrogène, le chlore, le fluor ou un reste alkyle en $C_1$ à $C_4$ et

$R^3$ et $R^4$ sont identiques ou différents et représentent le groupe

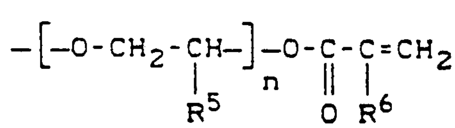

ou

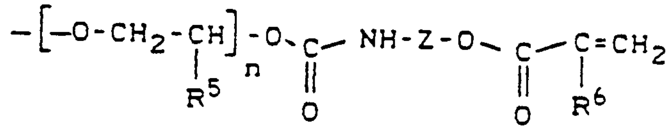

où

$R^5$ et $R^6$ sont identiques ou différents et représentent l'hydrogène ou le groupe méthyle,

Z est une chaîne alkylénique droite ou ramifiée en $C_2$ à $C_8$ et

n a des valeurs de 1 à 4.